(19) Europäisches Patentamt
European Patent Office
Office européen des brevets

(11) EP 3 811 946 A1

# (12) EUROPEAN PATENT APPLICATION
published in accordance with Art. 153(4) EPC

(43) Date of publication:
**28.04.2021 Bulletin 2021/17**

(21) Application number: **19808055.8**

(22) Date of filing: **22.05.2019**

(51) Int Cl.:
***A61K 31/519*** *(2006.01)* ***A61P 35/00*** *(2006.01)*

(86) International application number:
**PCT/CN2019/087945**

(87) International publication number:
**WO 2019/223716 (28.11.2019 Gazette 2019/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.05.2018 CN 201810499596**
**18.09.2018 CN 201811086544**
**11.10.2018 CN 201811182296**

(71) Applicant: **Jiangsu Hengrui Medicine Co., Ltd.**
**Lianyungang, Jiangsu 222047 (CN)**

(72) Inventors:
- **ZHANG, Lei**
  **Lianyungang, Jiangsu 222047 (CN)**
- **YANG, Changyong**
  **Lianyungang, Jiangsu 222047 (CN)**
- **LIAO, Cheng**
  **Lianyungang, Jiangsu 222047 (CN)**
- **ZHANG, Lianshan**
  **Lianyungang, Jiangsu 222047 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

## (54) USE OF CDK4/6 INHIBITOR IN COMBINATION WITH EGFR INHIBITOR IN THE PREPARATION OF MEDICAMENT FOR TREATING TUMOR DISEASES

(57) Provided in the present invention is a use of CDK4/6 inhibitor in combination with EGFR inhibitor in the preparation of a medicament for treating tumor diseases. In particular, provided in the invention is a use of a cyclin-dependent kinase 4 and 6 inhibitor (CDK4/6i) in combination with a human epidermal growth factor receptor inhibitor (EGFRi) for the preparation of a medicament for preventing or treating tumor diseases.

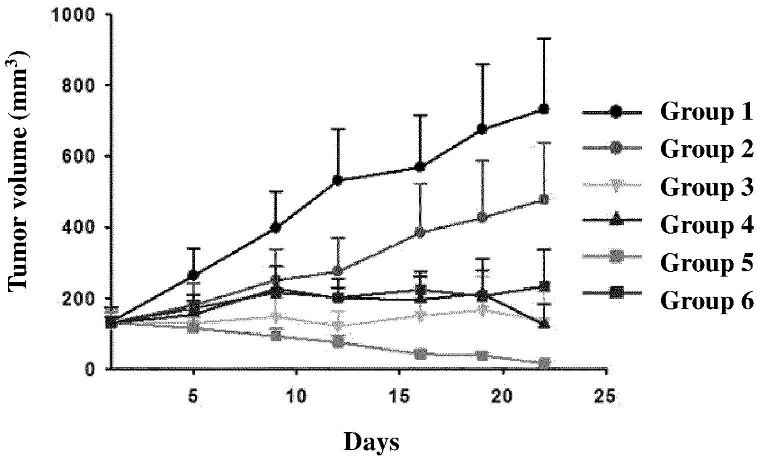


Figure 2



Printed by Jouve, 75001 PARIS (FR)

## Description

The present application claims the priority to Chinese Patent Application No. CN201810499596.2 filed on May 23, 2018, Chinese Patent Application No. CN201811086544.9 filed on September 18, 2018, and Chinese Patent Application No. CN201811182296.8 filed on October 11, 2018, the contents of which are incorporated herein by reference in their entirety.

## Technical Field

The invention pertains to the pharmaceutical field, which particularly relates to a use ofcyclin-dependent kinase 4 and 6 (CDK4/6) inhibitor (CDK4/6i) in combination with human epidermal growth factor receptor inhibitor (EGFRi) in the manufacture of a medicament for preventing or treating non-small cell lung cancer (NSCLC).

## Background arts

Lung cancer has become the leading cause of cancer deaths worldwide. In China, lung cancer ranks first in terms of cancer incidence and mortality. Although several newer generations of cytotoxic drugs and targeted therapies have been introduced in the past 20 years, patients with advanced lung cancer, especially those without known driver mutation genes, still have a poor survival prognosis. The advanced or metastatic lung cancer is still a fatal disease with a large number of unmet medical needs.

Non-small cell lung cancer (NSCLC) accounts for about 85% of all lung cancers. About 75% of NSCLC patients are already in the advanced stage when discovered, and the 5-year survival rate is quite low. There is still a great clinical need to choose an appropriate systemic treatment for the patients suffering from the advanced or metastatic NSCLC. NSCLC can be classified into squamous cell carcinoma and non-squamous cell carcinoma. Non-squamous cell carcinomas include adenocarcinoma, large cell carcinoma and other subtypes of cell carcinoma. Patients suffering from non-squamous cell carcinoma are further classified according to whether there is a driver mutation gene (EGFR mutation or ALK gene rearrangement).

EGFR (Epidermal Growth Factor Receptor) is a member of the erbB receptor family, which is transmembrane protein tyrosine kinase. By binding to its ligand, such as epidermal growth factor (EGF), EGFR can form a homodimer on the cell membrane or form a heterodimer with other receptors in the family, such as erbB2, erbB3, or erbB4. The formation of these dimers can cause the phosphorylation of key tyrosine residues in EGFR cells, thereby activating a number of downstream signaling pathways in cells. These intracellular signaling pathways play an important role in cell proliferation, survival and anti-apoptosis. Disorders of EGFR signal transduction pathways, including increased expression of ligands and receptors, EGFR gene amplification and mutation and the like, can promote malignant transformation of cells and play an important role in tumor cell proliferation, invasion, metastasis and angiogenesis. The over expression of EGFR has been reported in many human malignant diseases, including bladder cancer, brain tumors, head and neck cancer, pancreatic cancer, lung cancer, breast cancer, ovarian cancer, colon cancer, prostate cancer, and kidney cancer. In many cases, the over expression of EGFR is related to the poor prognosis of patients.

In addition, a large number of studies have found that tumors are related to cell cycle abnormalities. Most tumors have a large number of mutations of mitotic signaling protein/anti-mitotic signaling protein defects, genomic instability (GIN) and chromosome instability (CIN). These three basic cell cycle defects are directly or indirectly caused by the uncontrolled cyclin-dependent kinase (CDK). Cyclin B/CDK1, Cyclin A/CDK2, Cyclin E/CDK2, Cyclin D/CDK4, Cyclin D/CDK6 and other heterodimers (including CDK3 and CDK7) are important regulators of cell cycle progression. Some CDK inhibitors have been published, among which CDK4/6 inhibitors are abemaciclib, ribociclib, palbociclib, etc.

The prior art discloses a use of some CDK inhibitors in combination with EGFR kinase inhibitors in the manufacture of a medicament for treating non-small cell lung cancer, for example, the report "Synergistic combinations between the oral CDK inhibitor, seliciclib, and either EGFR inhibitors or DNA damaging agents in NSCLC" (AACR Annual Meeting--Apr 14-18, 2007*; Los Angeles, CA)* discloses that CDK inhibitor seliciclib in combination with EGFR kinase inhibitor has a synergistic effect in the treatment of NSCLC, wherein seliciclib is an inhibitor targeting CDK2, CDK7 and CDK9.

WO2017160568A discloses a use of necitumumab which is a recombinant human-derived IgG1 monoclonal antibody in combination with abemaciclib which is a cyclin-dependent kinase CDK4 and CDK6 inhibitor in the treatment of non-small cell lung cancer. References "PD 0332991, a selective cyclin D kinase 4/6 inhibitor, sensitizes lung cancer cells to treatment with epidermal growth factor receptor tyrosine kinase inhibitors" (Oncotarget. 2016 Dec 20; 7(51): 84951-84964) discloses that PD 0332991 (i.e., palbociclib) and Gefitinib are used to inhibit the growth of lung adenocarcinoma cell lines.

WO2014183520 provides an effective CDK4/6 inhibitor, the structure of which is represented by formula (I), and WO2016124067 discloses the hydroxyethyl sulfonate of the novel CDK4/6 inhibitor

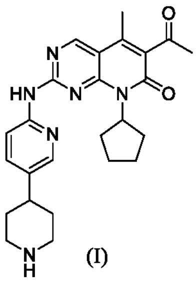

(I)

WO2016054987A discloses a 4-substituted-2-(*N*-(5-allylamido)phenyl)amino)pyrimidine derivative represented by formula (II), which has inhibitory activity against the EGFR-L858R mutant, the EGFR-T790M mutant and the mutant activated by exon 19 deletion, and can be used to treat diseases solely or partially mediated by the activity of EGFR mutants. WO2017161937A discloses the mesylate of the EGFR inhibitor represented by formula (II),

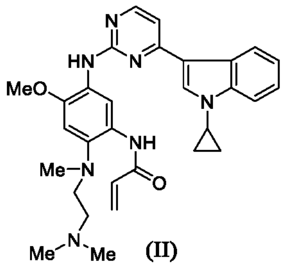

(II)

The present invention provides a noveluse of CDK4/6 inhibitor in combination with EGFR inhibitor in the manufacture of a medicament for preventing or treating non-small cell lung cancer, which exhibits good effects.

## Content of the present invention

The invention provides a use of CDK4/6 inhibitor in combination with EGFR inhibitor in the manufacture of a medicament for preventing or treating tumor disease.

The tumor disease of the present invention can be selected from the group consisting of breast cancer, ovarian cancer, prostate cancer, melanoma, brain tumor, esophageal cancer, stomach cancer, liver cancer, pancreatic cancer, colorectal cancer, lung cancer, kidney cancer, skin cancer, glioblastoma, neuroblastoma, sarcoma, liposarcoma, osteochondroma, osteoma, osteosarcoma, seminoma, testicular tumor, uterine cancer, head and neck tumor, multiple myeloma, malignant lymphoma, polycythemia vera, leukemia, thyroid tumor, ureteral tumor, bladder tumor, gallbladder cancer, cholangiocarcinoma or choriocarcinoma; preferably, non-small cell lung cancer.

The invention provides a use of CDK4/6 inhibitor in combination with EGFR inhibitor in the manufacture of a medicament for preventing or treating tumor disease, wherein the tumor disease is an EGFR mutation tumor disease.

The EGFR mutation tumor disease of the present invention is preferably non-small cell lung cancer, and preferably, the EGFR mutant is L858R EGFR mutant and/or T790M EGFR mutant.

In the optional embodiments, the non-small cell lung cancer of the present invention is squamous cell carcinoma and non-squamous cell carcinoma, preferably, non-phosphorous cell carcinoma, wherein the non-phosphorous cell carcinoma can be adenocarcinoma, large cell carcinoma and other subtypes of cell carcinoma.

In the optional embodiments, the CDK4/6 inhibitor can be abemaciclib, ribociclib, palbociclib, alvocidib, trilaciclib, voruciclib, AT-7519, G1T-38, FLX-925, INOC-005, G1T28-1, BPI-1178, gossypin, G1T30-1, GZ-38-1, P-276-00, staurosporine, R-547, PAN-1215, PD-0183812, AG-024322, NSC-625987, CGP-82996, PD-171851 or the compound represented by formula (I), the complex thereof or the pharmaceutically acceptable salt thereof, preferably abemaciclib, ribociclib, palbociclib, alvocidib or the compound represented by formula (I), the complex thereof or the pharmaceutically acceptable salt thereof, the most preferably the compound represented by formula (I), the complex thereof or the pharmaceutically acceptable salt thereof,

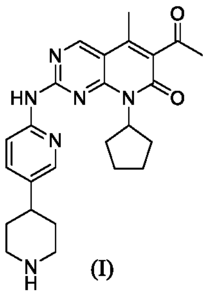

(I)

.

In the optional embodiments, the EGFR inhibitor can be osimertinib, gefitinib, erlotinib, olmutinib, icotinib, pyrotinib, vandetanib, brigatinib, dacomitinib, afatinib, neratinib, lapatinib, ABT-414, varlitinib, HLX-07, tesevatinib, theliatinib, epitinib succinate, S-222611, poziotinib, AST-2818, GNS-1480, mavelertinib, AP-32788, AZD-3759, nazartinib, Sym-013, allitinib tosylate, tarloxotinib bromide, CK-101, QL-1203, JNJ-61186372, SKLB-1028, TAS-121, Hemay-020, Hemay-022, NRC-2694-A, simotinib hydrochloride, SPH-1188-11, GR-1401, SYN-004, ABBV-221, MP-0274, GC-1118, BPI-15000, DBPR-112, Pirotinib, PB-357, lifirafenib, SCT-200, QLNC-120, agerafenib hydrochloride or the compound represented by formula (II), the stereoisomer thereof, the complex thereof or the pharmaceutically acceptable salt thereof, preferably olmutinib, afatinib, osimertinib, CK-101, erlotinib, icotinib, gefitinib or the compound represented by formula (II), the stereoisomer thereof, the complex thereof or the pharmaceutically acceptable salt thereof, the most preferably the compound represented by formula (II), the stereoisomer thereof, the complex thereof or the pharmaceutically acceptable salt thereof,

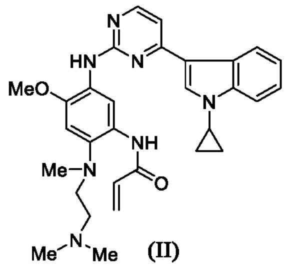

(II)

.

The pharmaceutically acceptable salt of the present invention can be hydrochloride, phosphate, hydrogen phosphate, sulfate, hydrogen sulfate, sulfite, acetate, oxalate, malonate, valerate, glutamate, oleate, palmitate, stearate, laurate, borate, *p*-toluenesulfonate, mesylate, hydroxyethyl sulfonate, maleate, malate, tartrate, benzoate, pamoate, salicylate, vanillate, mandelate, succinate, gluconate, lactobionate or lauryl sulfonate, etc.

In the preferable embodiments, the pharmaceutically acceptable salt of the compound represented by represented by formula (I) is hydroxyethyl sulfonate.

In the preferable embodiments, the pharmaceutically acceptable salt of the compound represented by represented by formula (II) is mesylate.

In the use of CDK4/6 inhibitor in combination with EGFR inhibitor in the manufacture of a medicament for preventing or treating tumor disease, the frequency of administration of the CDK4/6 inhibitor can be once a day, twice a day, or three times a day, and the frequency of administration of the EGFR inhibitor can be once a day, twice a day, or three times a day.

In the optional embodiments, the dose of the CDK4/6 inhibitor is 1-1000 mg, and the frequency of administration thereof can be once a day, twice a day, or three times a day, and the dose of the EGFR inhibitor is 1-1000 mg, and the frequency of administration thereof can be once a day, twice a day, or three times a day.

The dose of the CDK4/6 inhibitor of the present invention can be 5mg, 10mg, 15mg, 20mg, 25mg, 30mg, 35mg, 40mg, 45mg, 50mg, 55mg, 60mg, 65mg, 70mg, 75mg, 80mg, 85mg, 90mg, 100mg, 125mg, 150mg, 175mg, 200mg, 250mg, 300mg, 350mg, 400mg, 450mg, 500mg, 600mg, 700mg, 750mg, 800mg, 900mg or 1000mg.

The dose of the EGFR inhibitor of the present invention can be 1mg, 2.5mg, 5mg, 7.5mg, 10mg, 12.5mg, 15mg, 17.5mg, 20mg, 22.5mg, 25mg, 27.5mg, 30mg, 32.5mg, 35mg, 37.5mg, 40mg, 42.5mg, 45mg, 47.5mg, 50mg, 52.5mg, 55mg, 60mg, 65mg, 70mg, 75mg, 80mg, 85mg, 90mg, 95mg, 100mg, 105mg, 110mg, 120mg, 130mg, 140mg, 150mg, 160mg, 170mg, 180mg, 190mg, 200mg, 210mg, 220mg, 230mg, 240mg, 250mg, 260mg, 270mg, 280mg, 290mg, 300mg, 350mg, 400mg, 450mg, 500mg, 550mg, 600mg, 650mg, 700mg, 750mg, 800mg, 850mg, 900mg, 950mg or100mg.

In the preferable embodiments of the present invention, the CDK4/6 inhibitor is administered once a day, and

the dose thereof is preferably 25 mg, 50 mg, 75 mg, 100 mg, 125 mg, 150 mg or175 mg, the EGFR inhibitor is administered once a day, and the dose thereof is preferably 55 mg, 110mg, 220mg or260mg.

In the preferable embodiments of the present invention, the CDK4/6 inhibitor is the compound represented by formula (I), the complex thereof or the pharmaceutically acceptable salt thereof, which is administered once a day, and the dose thereof is preferably 25 mg, 50 mg, 75 mg, 100 mg, 125 mg, 150 mg or 175 mg, the EGFR inhibitor is the compound represented by formula (II), the stereoisomer thereof, the complex thereof or the pharmaceutically acceptable salt thereof, which is administered once a day, and the dose thereof is preferably 55 mg, 110mg, 220mg or 260mg.

In the optional embodiments of the present invention, the CDK4/6 inhibitor is the hydroxyethyl sulfonate of compound represented by formula (I), which is administered once a day, and the dose thereof can be 25 mg, 50 mg, 75 mg, 100 mg, 125 mg, 150 mg or 175 mg, the EGFR inhibitor is the mesylate of the compound represented by formula (II), which is administered once a day, and the dose thereof can be 55 mg, 110mg, 220mg or 260mg.

In the preferable embodiments, the CDK4/6 inhibitor is the hydroxyethyl sulfonate of compound represented by formula (I), which is administered once a day, and the dose thereof is 25 mg, and the EGFR inhibitor is the mesylate of the compound represented by formula (II), which is administered once a day, and the dose thereof is preferably 55 mg, 110mg, 220mg or 260mg.

In the preferable embodiments, the CDK4/6 inhibitor is the hydroxyethyl sulfonate of compound represented by formula (I), which is administered once a day, and the dose thereof is 50 mg, and the EGFR inhibitor is the mesylate of the compound represented by formula (II), which is administered once a day, and the dose thereof is preferably 55 mg, 110mg, 220mg or 260mg.

In the preferable embodiments, the CDK4/6 inhibitor is the hydroxyethyl sulfonate of compound represented by formula (I), which is administered once a day, and the dose thereof is 75 mg, and the EGFR inhibitor is the mesylate of the compound represented by formula (II), which is administered once a day, and the dose thereof is preferably 55 mg, 110mg, 220mg or 260mg.

In the preferable embodiments, the CDK4/6 inhibitor is the hydroxyethyl sulfonate of compound represented by formula (I), which is administered once a day, and the dose thereof is 100 mg, and the EGFR inhibitor is the mesylate of the compound represented by formula (II), which is administered once a day, and the dose thereof is preferably 55 mg, 110mg, 220mg or 260mg.

In the preferable embodiments, the CDK4/6 inhibitor is the hydroxyethyl sulfonate of compound represented by formula (I), which is administered once a day, and the dose thereof is 125 mg, and the EGFR inhibitor is the mesylate of the compound represented by formula (II), which is administered once a day, and the dose thereof is preferably 55 mg, 110mg, 220mg or 260mg.

In the preferable embodiments, the CDK4/6 inhibitor is the hydroxyethyl sulfonate of compound represented by formula (I), which is administered once a day, and the dose thereof is 150 mg, and the EGFR inhibitor is the mesylate of the compound represented by formula (II), which is administered once a day, and the dose thereof is preferably 55 mg, 110mg, 220mg or 260mg.

In the preferable embodiments, the CDK4/6 inhibitor is the hydroxyethyl sulfonate of compound represented by formula (I), which is administered once a day, and the dose thereof is 175 mg, and the EGFR inhibitor is the mesylate of the compound represented by formula (II), which is administered once a day, and the dose thereof is preferably 55 mg, 110mg, 220mg or 260mg.

In the preferable embodiments of the present invention, the weight ratio of the CDK4/6 inhibitor to the EGFR inhibitor is 0.001:1-1000:1, preferably, 0.01:1-100:1, the most preferably, 0.05:1-50:1, specifically can be 500:1, 400:1, 300:1, 200:1, 100:1, 50:1, 25:1, 12.5:1, 10:1, 8:1, 6:1, 5: 1, 3.18:1, 2.72:1, 2.27:1, 2:1, 1.81:1, 1.59:1, 1.36:1, 1.14:1, 1:1, 1:1.1, 1:1.5, 1:1.26, 1:1.47, 1:1.49, 1:1.73, 1:1.76, 1:2, 1:2.08, 1:2.2, 1:2.6, 1:2.93, 1:3.47, 1:3.5, 1:4.4, 1: 5, 1:5.2, 1:7.5, 1:8.8, 1:10, 1:10.4, 1:12.5, 1:15, 1:20, 1:25, 1:30, 1:50, 1:75, 1:100, 1:125, 1:150, 1:200, 1:250, 1:300, 1:400, 1:500, 1:600, 1:700 or 1:800.

The administration of the combination of the present invention can be oral administration, parenteral administration or transdermal administration, wherein the parenteral administration includes but not limited to intravenous injection, subcutaneous injection and intramuscular injection, preferably oral administration.

In the embodiments of the present invention, the combination optionally further contains other components, and the other components include but are not limited to other drugs for treating tumor diseases.

The present invention also provides a method for treating tumor disease, which comprises administering an effective amount of the CDK4/6 inhibitor and an effective amount of the EGFR inhibitor to a patient.

The present invention also provides a pharmaceutical composition, which comprises the CDK4/6 inhibitor, the EGFR inhibitor, and one or more than one pharmaceutical carriers, excipients or diluents. The pharmaceutical composition can be formulated as any pharmaceutically acceptable dosage form. For example, it can be formulated as tablets, capsules, pills, granules, solutions, suspensions, syrups, injections (including injection liquid, sterile powders for injection and concentrated solutions for injection), suppositories, inhalants or spray agents.

The pharmaceutical composition comprising the CDK4/6 inhibitor and the EGFR inhibitor of the present invention

can be administered either alone or in combination with one or more than one therapeutic agents.

The present invention also provides a pharmaceutical kit which is in the use of the medicament for treating tumor disease, wherein the pharmaceutical composition comprising the CDK4/6 inhibitor and the EGFR inhibitor of the present invention is packaged.

In the present invention, the CDK4/6 inhibitor is administered in combination with the EGFR inhibitor, thereby enhancing the use of the medicament for tumor disease and improving the therapeutic effect.

The expression "in combination with" in the present invention is a mode of administration, which means that at least one dose of the CDK4/6 inhibitor and at least one dose of the EGFR inhibitor are administered within a certain period of time, wherein both of the two substances exhibit pharmacological effects. The period of time can be within one administration cycle, preferably within 4 weeks, within 3 weeks, within 2 weeks, within 1 week, or within 24 hours. The CDK4/6 inhibitor and the EGFR inhibitor can be administered simultaneously or sequentially. This period of time includes the treatment in which the CDK4/6 inhibitor and the EGFR inhibitor are administered via the same route or different routes.

The term "effective amount" refers to an amount of a drug effective to treat a disease or disorder in a mammal. In the case of cancer, the therapeutically effective amount of the drug may reduce the number of cancer cells, reduce the tumor size, inhibit (i.e., slow to some extent and preferably stop) cancer cell infiltration into peripheral organs, inhibit (i.e., slow to some extent and preferably stop) tumor metastasis, inhibit, to some extent, tumor growth, and/or relieve to some extent one or more than one symptoms associated with the disorder. Depending on the extent to which the drug may prevent the growth of and/or kill existing cancer cells, it may be cytostatic and/or cytotoxic. For cancer therapy, efficacy *in vivo* can, for example, be measured by assessing the duration of overall survival (OS), duration of progression free survival (PFS), the response rates (RR), duration of response, and/or quality of life.

## Brief description of the drawings

Figure 1 shows the effect of drug A in combination with drug B on the body weight of nude mice.

Figure 2 shows the effect of drug A in combination with drug B on the tumor volume of the subcutaneously transplanted tumor model of human lung cancer cell NCI-H1975.

Figure 3 shows the effect of drug A in combination with drug B on the relative tumor volume of the subcutaneously transplanted tumor model of human lung cancer cell NCI-H1975.

## Detailed description of the preferred embodiment

Hereinafter, the present invention will be explained in more detail with reference to the embodiments. The embodiments of the present invention are only used to illustrate the technical solutions of the present invention, and do not limit the substantial and scope of the present invention.

**Example 1** Evaluation of the hydroxyethyl sulfonate of the compound represented by formula (I) (drug A) in combination with the mesylate of compound represented by formula (II) (drug B) on the proliferation inhibition of human non-small cell lung adenocarcinoma cells (NCI-H1975).

### 1. Experimental materials

Human lung adenocarcinoma cells NCI-H1975 (carrying the EGFR21 exon L858R mutation and the 20 exon T790M mutation) were incubated by the Cancer and Endocrine Pharmacology Laboratory, Department of Pharmacy, Zhejiang University and stored in liquid nitrogen;
Drug A was prepared according to the method disclosed in WO2016124067A;
Drug B was prepared according to the method disclosed in WO2017161937A.

### 2. Experimental method

NCI-H1975 cells with good growth status were seeded in 96-well plates, and after overnight adherent growth, they were administrated with test substances in different concentrations respectively, in triple replicate for each concentration. The final concentration of the test substance was set to 0.125, 0.25, 0.5, 1 and 2μM for the hydroxyethyl sulfonate of the compound represented by formula (I), and was set to 5 and 10 nM for the compound represented by formula (II). After NCI-H1975 cells were treated with the test substances alone or in combination for 7 days, the *in vitro* proliferation

inhibitory effect of the test substances on NCI-H1975 cells was detected based on the SRB method.

**3. Results processing**

According to the OD value measured by the microplate reader, the inhibition rate was calculated based on the following formula:

$$\text{Inhibition rate }(\%)=(1\text{-}OD_{\text{administration}}/OD_{\text{control}})\times 100\%$$

**4. Experimental results**

The hydroxyethyl sulfonate of the compound represented by formula (I) can inhibit the growth of NCI-H1975 cells in a concentration-dependent manner within a concentration range of 0.125-2 μM. Drug A in combination with 5 nM or 10 nM drug B can increase the proliferation inhibition rate of NCI-H1975 cells relative to the two drugs used alone. See Table 1 for details.

Table 1

| Drug A | | Drug B | | In Combination |
|---|---|---|---|---|
| Concentration (μM) | Inhibition rate (%) | Concentration (nM) | Inhibition rate (%) | Inhibition rate (%) |
| 0.125 | 46.3 | 5 | 50.3 | 77.3 |
| 0.25 | 50.0 | | | 81.3 |
| 0.5 | 56.0 | | | 84.3 |
| 1 | 62.4 | | | 85.5 |
| 2 | 73.5 | | | 90.6 |
| 0.125 | 46.3 | 10 | 53.8 | 82.2 |
| 0.25 | 50.0 | | | 82.0 |
| 0.5 | 56.0 | | | 83.6 |
| 1 | 62.4 | | | 85.0 |
| 2 | 73.5 | | | 92.3 |

**Example 2** Evaluation of the experimental therapeutic effect of drug A in combination with drug B on human lung cancer NCI-H1975 transplanted tumor model of nude mice

**1. Experimental materials**

Experimental animals: nude mice, female; 8 weeks, 24 vaccinated, 18 actually used, provided by Shanghai Xipuer-Bikai Experimental Animal Co., Ltd.;
Test drugs: Drug A and Drug B are the same as those used in Example 1, the control drug gefitinib was provided by the Department of Pharmacy, Zhejiang University.

**2. Experimental method**

1) Dose and frequency of administration

Gefitinib: 30 mg/kg, once a day, Drug A: 100 (50) mg/kg, once a day, Drug B: 5 mg/kg, once a day.

2) Drug formulation

6 mg gefitinib was weighed, added with an appropriate amount of 0.5% CMC-Na, grinded evenly, transferred to an EP tube, added with 0.5% CMC-Na to 2 mL, and mixed evenly to obtain a solution of gefitinib with a concentration of 3 mg/mL, which was freshly prepared before use.

20 mg drug A was weighed, added with an appropriate amount of 0.5% MC, grinded evenly, transferred to an EP tube, added with 0.5% MC to 2 mL, and mixed evenly to obtain a solution of drug A with a concentration of 10 mg/mL, which was freshly prepared before use.

1 mg drug B was weighed, added with an appropriate amount of 0.5% MC, grinded evenly, transferred to an EP tube, added with 0.5% MC to 2 mL, and mixed evenly to obtain a solution of drug B with a concentration of 0.5 mg/mL, which was freshly prepared before use.

3) Model establishment

$1\times10^7$ human lung cancer cells NCI-H1975 cells were injected into the armpits of nude mice. After the tumor grew to a suitable size, the NCI-H1975 mouse tumor was removed and placed in a container filled with saline. The surface blood vessels were peeled off and the necrotic area was discarded. The tumor was then cut into 1-2 mm3, and the tumor was inserted into the left armpit of the nude mouse with a trocar. After the tumor grew to an average volume of 50-150 $mm^3$, 18 mice were divided into 6 groups (3 in each group), namely the negative control group (Control), the group of "gefitinib at a dose of 30 mg/kg", the group of "drug A at a dose of 100(50) mg/kg", the group of "drug B at a dose of 5 mg/kg", the group of "drug A in combination with drug B" and the group of "drug A in combination with gefitinib".

4) Specific procedure

All nude mice were administered by intragastric administration, the administration volume was 10 mL/kg, and the tumor volume was weighed and measured twice a week. The administration cycle was 21 days. On the $22^{nd}$ day, the mice were weighed and the tumor volume was measured. The mice were then sacrificed and the tumor was weighed. The relative tumor volume (RTV), relative tumor growth rate (T/C) and tumor inhibition percentage (IR) were calculated and the statistical test (SPSS test) was performed.

Calculation formula shown as follows:

(1) TV (tumor volume) = $1/2\times a\times b^2$, wherein, a and b represent the length and width of the tumor, respectively;

(2) RTV (relative tumor volume) = $V_t/V_0$, wherein, Vo is the tumor volume measured at grouping (i.e., do), and $V_t$ is the tumor volume at each measurement;

(3) T/C (%) = $T_{RTV}/C_{RTV}\times100\%$, wherein $T_{RTV}$ is the RTV of the treatment group, and $C_{RTV}$ is the RTV of the control group;

(4) IR (%) = $(1- TW_t/TW_c)\times100\%$, wherein $TW_t$ is the tumor weight of the treatment group, $TW_c$ is the tumor weight of the control group.

**3. Experimental results**

At the end of the experiment, compared with the negative control group, the body weight of mice in each administration group has no significant change. (See Table 2 and Figure 1 for details).

Table 2. The effect of drug A in combination with drug B on the body weight of nude mice ($\bar{x} \pm SE$)

| Group | Drug | Dose (mg/kg) | Animal number | | Average body weight (g) | |
|---|---|---|---|---|---|---|
| | | | D1 | D22 | D1 | D22 |
| 1 | Negative control | - | 3 | 3 | 22.7±0.3 | 24.7±0.4 |
| 2 | Gefitinib | 30 | 3 | 3 | 21.8±0.6 | 24.5±0.8 |
| 3 | Drug A | 100(50) | 3 | 3 | 22.8±0.3 | 23.5±0.7 |
| 4 | Drug B | 5 | 3 | 3 | 22.8±0.7 | 21.8±1.4 |
| 5 | Drug A+Drug B | 100(50)+5 | 3 | 3 | 22.3±0.4 | 20.4±0.9 |
| 6 | Drug A + Gefitinib | 100(50)+30 | 3 | 3 | 20.7±0.6 | 22.5±0.8 |

From Day 1 to Day 12, the dose of drug A in the group of "drug A"and the group of "combination" was 100 mg/kg. From Day 13 to Day 21, the dose was changed to 50 mg/kg. Compared with the negative control, there is no significant difference in each group.

At the end of the experiment, compared with the tumor volume of the negative control group being 732±200 $mm^3$, the tumor volumes of group 2 to group 6 are 477±162 $mm^3$, 136±83 $mm^3$, 125±58 $mm^3$, 17±3 $mm^3$ and 233±104 $mm^3$, respectively (see Table 3 and Figure 2 for details). Compared with the RTV value of the negative control group being 4.77±0.48, the RTV values of group 2 to group 6 are 3.18±0.65, 0.81±0.33, 1.25±0.53, 0.18±0.07 and 1.71±0.71, respectively; and T/C values are 66.81 %, 17.00%, 26.14%, 3.86% and 35.91%, respectively (see Table 3 and Figure 3 for details).

Table 3. Effect of drug A in combination with drug B on subcutaneous transplanted tumor ($\bar{x}$ ± *SE)*

| Group | Tumor volume ($mm^3$) | | RTV | T/C(%) |
|---|---|---|---|---|
| | D1 | D22 | | |
| 1 | 133±35 | 732±200 | 4.77±0.48 | - |
| 2 | 130±41 | 477±162 | 3.18±0.65 | 66.81 |
| 3 | 130±36 | 136±83 | 0.81±0.33 | 17.00 |
| 4 | 130±44 | 125±58 | 1.25±0.53 | 26.14 |
| 5 | 130±29 | 17±3 | 0.18±0.07 | 3.86 |
| 6 | 130±17 | 233±104 | 1.71±0.71 | 35.91 |

At the end of the experiment, compared with the tumor weight of the the negative control group being 0.5958±0.1900 g, the tumor weights of group 2 to group 6 are 0.4097±0.1605 g, 0.1269±0.0839 g, 0.1091±0.0621 g, 0.0061±0.0002 g and 0.2153±0.1119 g, respectively; and IR are 31.24%, 78.69%, 81.69%, 98.98% and 63.86%, respectively (see Table 4 for details).

Table 4. Effect of drug A in combination with drug B on the weight of subcutaneous transplanted tumor ($\bar{x}$ ± *SE*)

| Group | Tumor weight (g) | IR (%) |
|---|---|---|
| 1 | 0.5958±0.1900 | - |
| 2 | 0.4097±0.1605 | 31.24 |
| 3 | 0.1269±0.0839 | 78.69 |
| 4 | 0.1091±0.0621 | 81.69 |
| 5 | 0.0061±0.0002 | 98.98 |
| 6 | 0.2153±0.1119 | 63.86 |

From Day 1 to Day 12, the dose of drug A in the group of "drug A" and the group of "combination" was 100 mg/kg. From Day 13 to Day 21, the dose was changed to 50 mg/kg.

Under the experimental conditions, drug A at a dose of 100 (50) mg/kg in combination with drug B at a dose of 5 mg/kg (q.d., 21 days in total) can inhibit the growth of human lung cancer NCI-H1975 transplanted tumors in nude mice, and the effect is better than that of drug A in combination with gefitinib.

## Claims

1. A use of the combination of CDK4/6 inhibitor and EGFR inhibitor in the manufacture of a medicament for preventing or treating tumor disease.

2. The use as defined in claim 1, wherein the tumor disease is selected from the group consisting of breast cancer, ovarian cancer, prostate cancer, melanoma, brain tumor, esophageal cancer, stomach cancer, liver cancer, pancreatic cancer, colorectal cancer, lung cancer, kidney cancer, skin cancer, glioblastoma, neuroblastoma, sarcoma, liposarcoma, osteochondroma, osteoma, osteosarcoma, seminoma, testicular tumor, uterine cancer, head and neck tumor, multiple myeloma, malignant lymphoma, polycythemia vera, leukemia, thyroid tumor, ureteral tumor, bladder tumor, gallbladder cancer, cholangiocarcinoma or choriocarcinoma; preferably, non-small cell lung cancer.

3. The use as defined in claim 1, wherein the tumor disease is an EGFR mutation tumor disease.

4. The use as defined in claim 3, wherein the EGFR mutation tumor disease is non-small cell lung cancer, the EGFR

mutant is preferably L858R EGFR mutant and/or T790M EGFR mutant.

5. The use as defined in claim 2, wherein the non-small cell lung cancer is squamous cell carcinoma or non-squamous cell carcinoma, preferably, non-phosphorous cell carcinoma.

6. The use as defined in any one of claims 1 to 5, wherein the CDK4/6 inhibitor is selected from the group consisting of abemaciclib, ribociclib, palbociclib, alvocidib, trilaciclib, voruciclib, AT-7519, G1T-38, FLX-925, INOC-005, G1T28-1, BPI-1178, gossypin, G1T30-1, GZ-38-1, P-276-00, staurosporine, R-547, PAN-1215, PD-0183812, AG-024322, NSC-625987, CGP-82996, PD-171851 or the compound represented by formula (I), the complex thereof and the pharmaceutically acceptable salt thereof, preferably abemaciclib, ribociclib, palbociclib, alvocidib, the compound represented by formula (I), the complex thereof and the pharmaceutically acceptable salt thereof, the most preferably the compound represented by formula (I), the complex thereof and the pharmaceutically acceptable salt thereof,

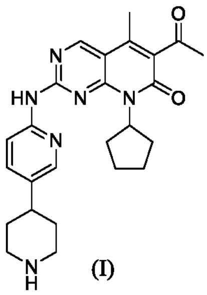

(I)

.

7. The use as defined in any one of claims 1 to 5, wherein the EGFR inhibitor is selected from the group consisting of osimertinib, gefitinib, erlotinib, olmutinib, icotinib, pyrotinib, vandetanib, brigatinib, dacomitinib, afatinib, neratinib, lapatinib, ABT-414, varlitinib, HLX-07, tesevatinib, theliatinib, epitinib succinate, S-222611, poziotinib, AST-2818, GNS-1480, mavelertinib, AP-32788, AZD-3759, nazartinib, Sym-013, allitinib tosylate, tarloxotinib bromide, CK-101, QL-1203, JNJ-61186372, SKLB-1028, TAS-121, Hemay-020, Hemay-022, NRC-2694-A, simotinib hydrochloride, SPH-1188-11, GR-1401, SYN-004, ABBV-221, MP-0274, GC-1118, BPI-15000, DBPR-112, Pirotinib, PB-357, lifirafenib, SCT-200, QLNC-120, agerafenib hydrochloride, the compound represented by formula (II), the stereoisomer thereof, the complex thereof and the pharmaceutically acceptable salt thereof, preferably olmutinib, afatinib, osimertinib, CK-101, erlotinib, icotinib, gefitinib, the compound represented by formula (II), the stereoisomer thereof, the complex thereof and the pharmaceutically acceptable salt thereof, the most preferably the compound represented by formula (II), the stereoisomer thereof, the complex thereof and the pharmaceutically acceptable salt thereof,

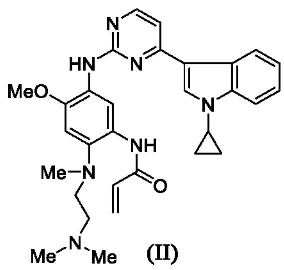

(II)

.

8. The use as defined in claim 6, wherein the pharmaceutically acceptable salt of the compound represented by represented by formula (I) is hydroxyethyl sulfonate.

9. The use as defined in claim 7, wherein the pharmaceutically acceptable salt of the compound represented by represented by formula (II) is mesylate.

10. The use as defined in any one of claims 1 to 9, wherein the dose of the CDK4/6 inhibitor is 1-1000 mg, and the frequency of administration thereof is once a day, twice a day, or three times a day, and the dose of the EGFR inhibitor is 1-1000 mg, and the frequency of administration thereof is once a day, twice a day, or three times a day.

11. The use as defined in claim 10, wherein the weight ratio of the CDK4/6 inhibitor to the EGFR inhibitor is 0.001:1-1000:1,

preferably 0.01:1-100:1, the most preferably 0.05:1-50:1.

**12.** The use as defined in claim 10, wherein the CDK4/6 inhibitor is administered once a day, and the dose thereof is 25 mg, 50 mg, 75 mg, 100 mg, 125 mg, 150 mg and 175 mg, and the EGFR inhibitor is administered once a day, and the dose thereof is 55 mg, 110mg, 220mg and 260mg.

**13.** A pharmaceutical composition, which comprises the CDK4/6 inhibitor and the EGFR inhibitor as defined in any one of claims 1 to 12, and one or more than one pharmaceutical carriers, excipients or diluents.

**14.** A pharmaceutical kit, which comprises the pharmaceutical composition as defined in claim 13.

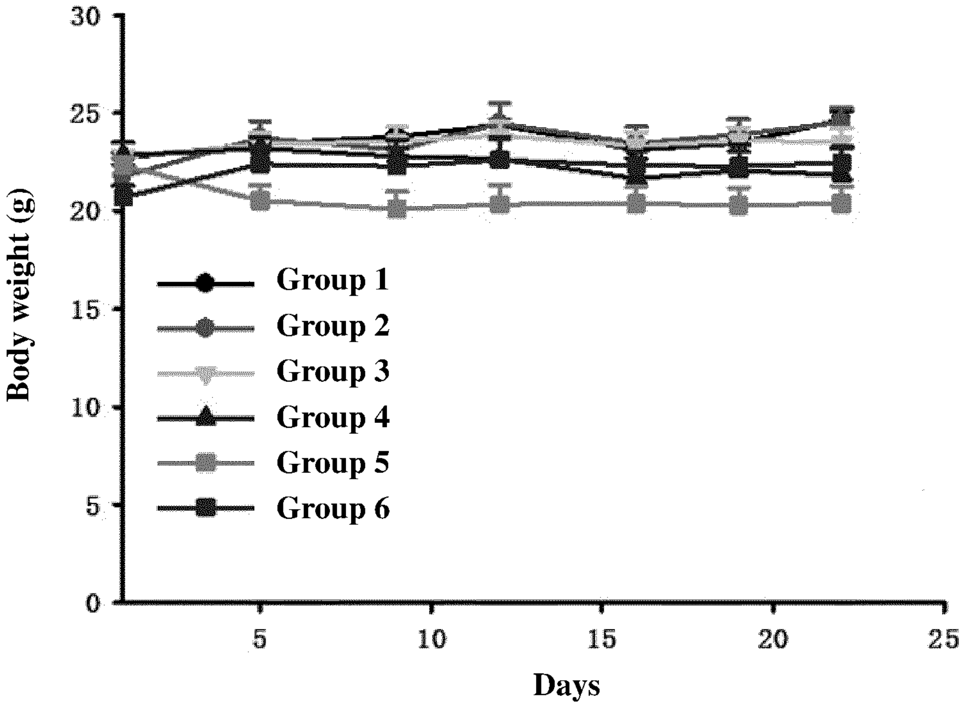


Figure 1

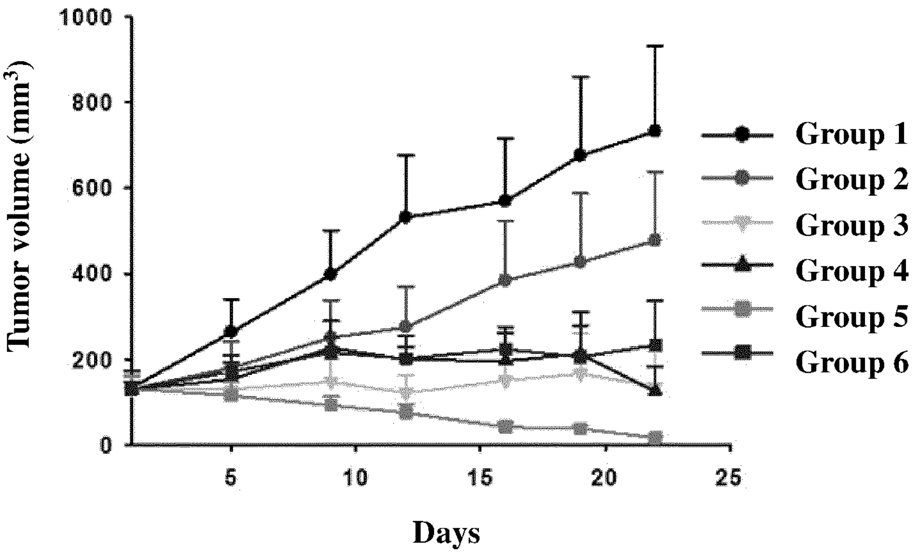


Figure 2

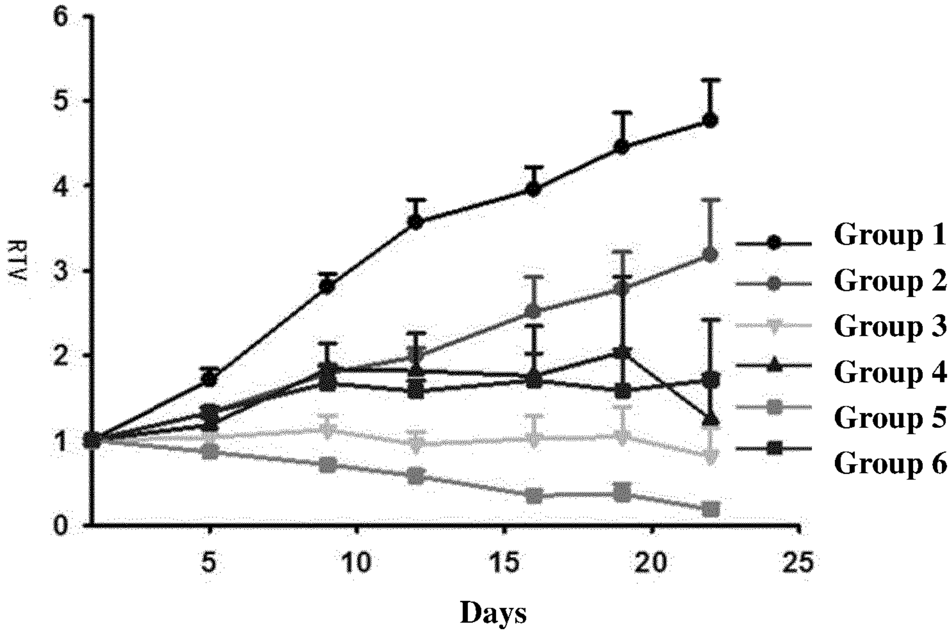
6
5
4
3
2
1
0
RTV
5
10
15
20
25
Days
Group 1
Group 2
Group 3
Group 4
Group 5
Group 6

Figure 3

**INTERNATIONAL SEARCH REPORT**

International application No. **PCT/CN2019/087945**

**A. CLASSIFICATION OF SUBJECT MATTER**

A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K,A61P

Documentation searched other than minimum documentation to the extent that such documents are included in the fields searched

Electronic data base consulted during the international search (name of data base and, where practicable, search terms used)

DWPI, SIPOABS, CNABS, CNMED, CNTXT, CNKI, REGISTRY, CAPLUS, MEDLINE, VEN, ISI-WEB OF SCIENCE, 细胞周期蛋白依赖性激酶, 表皮生长因子受体抑制剂, 非小细胞肺癌, CDK, EGFR, NSCLC

**C. DOCUMENTS CONSIDERED TO BE RELEVANT**

| Category* | Citation of document, with indication, where appropriate, of the relevant passages | Relevant to claim No. |
|---|---|---|
| X | CN 101678029 A (CYCLACEL LIMITED) 24 March 2010 (2010-03-24)<br>claims 1-3, 11-13, 24 and 42-44 | 1-5, 10-14 |
| Y | CN 101678029 A (CYCLACEL LIMITED) 24 March 2010 (2010-03-24)<br>claims 1-3, 11-13, 24 and 42-44 | 6-14 |
| Y | WO 2016124067 A (JIANGSU HENGRUI MEDICINE CO., LTD.) 11 August 2016 (2016-08-11)<br>description, pages 1-2, and claim 1 | 6, 8, 10-14 |
| Y | WO 2017161937 A (JIANGSU HANSOH PHARMACEUTICAL GROUP CO., LTD.) 28 September 2017 (2017-09-28)<br>abstract, and description, pages 1-2 and 8 | 7, 9-14 |

☐ Further documents are listed in the continuation of Box C.  ☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **22 July 2019** | **29 August 2019** |
| Name and mailing address of the ISA/CN<br>**China National Intellectual Property Administration**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/087945**

| Patent document cited in search report | | | Publication date (day/month/year) | Patent family member(s) | | | Publication date (day/month/year) |
|---|---|---|---|---|---|---|---|
| CN | 101678029 | A | 24 March 2010 | JP | 2010523536 | A | 15 July 2010 |
| | | | | CN | 101678029 | B | 18 July 2012 |
| | | | | JP | 5514099 | B2 | 04 June 2014 |
| | | | | EP | 2139486 | A1 | 06 January 2010 |
| | | | | WO | 2008122779 | A1 | 16 October 2008 |
| | | | | US | 9173938 | B2 | 03 November 2015 |
| | | | | US | 2010143350 | A1 | 10 June 2010 |
| WO | 2016124067 | A | 11 August 2016 | AU | 2016214897 | A1 | 10 August 2017 |
| | | | | JP | 2018503634 | A | 08 February 2018 |
| | | | | US | 2018230147 | A1 | 16 August 2018 |
| | | | | CA | 2974021 | A1 | 11 August 2016 |
| | | | | CN | 106661025 | A | 10 May 2017 |
| | | | | EP | 3255046 | A1 | 13 December 2017 |
| | | | | MX | 2017009270 | A | 11 October 2017 |
| | | | | US | 10160759 | B2 | 25 December 2018 |
| | | | | EP | 3255046 | A4 | 10 October 2018 |
| | | | | US | 2018009804 | A1 | 11 January 2018 |
| | | | | HK | 1232867 | A0 | 19 January 2018 |
| | | | | US | 10030018 | B2 | 24 July 2018 |
| WO | 2017161937 | A | 28 September 2017 | EP | 3434673 | A1 | 30 January 2019 |
| | | | | KR | 20180131572 | A | 10 December 2018 |
| | | | | JP | 2019509290 | A | 04 April 2019 |
| | | | | US | 2019077791 | A1 | 14 March 2019 |
| | | | | AU | 2016399168 | A1 | 25 October 2018 |
| | | | | TW | 201734006 | A | 01 October 2017 |
| | | | | CA | 3016092 | A1 | 28 September 2017 |
| | | | | CN | 108884072 | A | 23 November 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201810499596
- CN 201811086544
- CN 201811182296
- WO 2017160568 A
- WO 2014183520 A
- WO 2016124067 A
- WO 2016054987 A
- WO 2017161937 A


### Non-patent literature cited in the description


- Synergistic combinations between the oral CDK inhibitor, seliciclib, and either EGFR inhibitors or DNA damaging agents in NSCLC. *AACR Annual Meeting,* 14 April 2007
- PD 0332991, a selective cyclin D kinase 4/6 inhibitor, sensitizes lung cancer cells to treatment with epidermal growth factor receptor tyrosine kinase inhibitors. *Oncotarget,* 20 December 2016, vol. 7 (51), 84951-84964